# EUROPEAN PATENT SPECIFICATION

(11) **EP 3 880 137 B1**
(45) Date of publication and mention of the grant of the patent: **16.07.2025**
(21) Application number: 19884949.9
(22) Date of filing: 15.11.2019
(51) Int. Cl.: A61F 7/00, A61N 1/16, A61F 13/02, A61F 7/02, A61F 13/00

(54) **KINESIOLOGY TAPE**
KINESIOLOGIEBAND
BANDE DE KINÉSIOLOGIE

(30) Priority: 16.11.2018 FI 20185968
(43) Date of publication of application: 22.09.2021
(73) Proprietor: Oy Sda Finland Ltd, 01720 Vantaa (FI)
(72) Inventor: TUOMINEN, Vesa, 01720 Vantaa (FI)
(74) Representative: Kolster Oy Ab
(86) International application number: PCT/FI2019/050816
(87) International publication number: WO 2020/099724

(56) References cited:
- WO-A1-2015/021359
- WO-A1-2015/171467
- WO-A1-2017/077410
- WO-A1-94/19519
- WO-A2-2012/033932
- CN-U- 203 280 157
- CN-U- 206 214 260
- CN-U- 206 214 260
- US-A1- 2004 044 341
- US-A1- 2016 076 170
- US-A1- 2016 113 819
- US-A1- 2018 289 530
- US-A1- 2018 310 883

## Description

### Background of the invention

The present invention relates to kinesiology tape.

From prior art, kinesiology tapes are known for treating and preventing sports injuries. The thin tape is elastic and conforming to the skin and comprises an adhesive on one side thereof to fasten the tape to a desired place on the skin. The base material of the tape may be cotton, polyester, or nylon, which may comprise an elastomer to provide the structure with flexibility. On a surface of the structure there is an adhesive to fasten the tape in its stretched form to a body part being supported or treated. The tape may stay in place for several days. It can also withstand moisture.

Prior art document WO2017077410A1 discloses a kinesiology tape with a conductive heating element.

The tape may also be used to support joints and to avoid their potentially harmful extreme positions.

By means of the tape, muscle mobility may be enhanced. By means of the tape, blood circulation may be normalized. Swelling of an injured place may be reduced with the tape.

The tape is also suitable for treating operation scars. The use of the tape adds to muscular strength and lessens muscular pain. It is suitable for preventing sports-related injuries and treating injuries already taken place. Its use improves lymph flow of tissues and reduces swelling caused by tissue damage. The use of the tape normalises blood circulation.

### Brief description of the invention

To further develop kinesiology tape, this application proposes the use of an electrically conductive structure in connection with the normal tape structure.

Research has found out that electricity and a magnetic field reduce the viscosity of blood and tissue fluid. By using an electrically conductive layer or separate electrically conductive threads in the tape, or by saturating the base material, such as cotton or nylon, with electrically conductive particles, a healing effect is achieved by the tape for the damaged tissue area.

By the use of conductive thread structures wound in different directions in adjacent threads of the tape, a structure that not only protects a tissue against electromagnetic radiation but also a thermal effect that treats a damaged place is achieved. By using threads, a two-fold effect is consequently achieved: one that protects against electromagnetic radiation and produces a thermal effect.

The invention is defined in appended independent claim 1, preferred embodiments are described in the dependent claims.

The kinesiology tape according to the invention has a flexible carrier layer, such as a cotton layer, nylon layer, or polyester layer. The carrier layer comprises an associated elastomer which provides the structure with resilience and flexibility.

On a one surface of the carrier layer there is an adhesive used to fasten the structure on the skin in connection with the treated or supported place.

According to an embodiment, the tape is treated with a substance, such as a liquid or paint, comprising metal particles.

In an embodiment, electrically conductive threads are used in connection with the structure.

In an embodiment, the threads are wound around their winding axes so that in adjacent threads the winding direction is clockwise on one thread and counterclockwise on the adjacent thread. The winding axes are straight, and a distance E between them is in the range of 0.5 to 10 mm.

With the use of wound threads, the benefit is achieved that they are flexible. This way the elasticity of the kinesiology tape is realized.

As an adhesive, the tape may utilize an acrylic adhesive.

Electric conductivity may also be realized by using electrically conductive gel on the tape, or cotton fabric saturated with electrically conductive liquid in liquid form. On the surface, a thin, electrically conductive coating of paint may also be used.

The solution according to the invention is excellently suited to rehabilitation of sports injuries and alleviating muscular pain.

In an embodiment of the invention, a tape of a suitable shape may be cut as the injury dictates from a rectangular sheet. It may be placed around a knee, for example.

Kinesiology tape may be on a roll or as sheets, which is a more appropriate form from the viewpoint of posting.

The kinesiology tape according to the invention is characterized by what is disclosed in the claims.

### Brief description of the figures

In the following, the invention is described with reference to the described embodiment to which the invention is not intended to be exclusively restricted.

List of figures:
Figure 1 shows kinesiology tape in its most common application.
Figure 2 shows an embodiment where kinesiology tape is provided with longitudinal, electrically well conductive wound threads.
Figure 3 shows an embodiment where the electrically conductive threads of kinesiology tape are wound i.e. coiled, and in which the winding or coiling directions of adjacent threads are different.
Figure 4 shows how threads are introduced as closed loops and the grounding thereof.
Figure 5 shows an embodiment of the tape, in which there are cutting lines marked on the tape.
Figure 6 show unwinding kinesiology tape from a roll.

### Detailed description of the invention

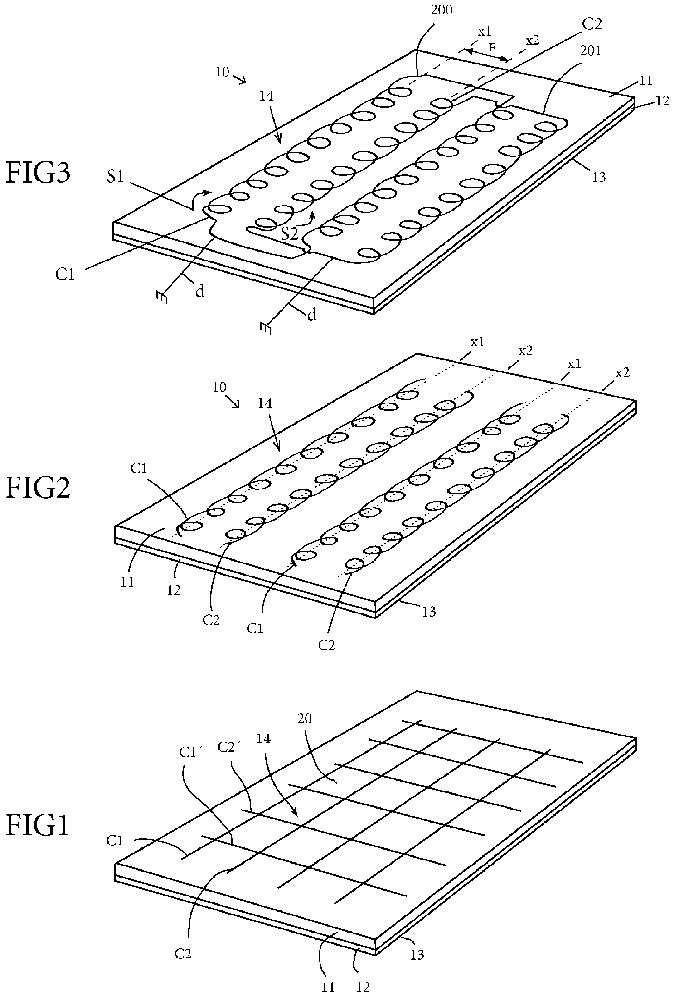

Figure 1 shows an embodiment where kinesiology tape 10, as unwound from a roll R, comprises a carrier layer 11 and thereon an elastomer layer 12 providing elasticity. In connection with the carrier layer 11 there is placed an electrically conductive structure 14. It is possible to lay the electrically conductive structure elsewhere, too, such as in connection with the elastomer layer. The electrically conductive structure 14 is formed of conductive threads C1, C2, C1', C2'... advantageously knitted in connection with the carrier layer 11. Electrically well conducting threads C1, C2, C1', C2'... are knitted on the surface, which may be parallel to each other or crosswise, and they form a grid or mesh 20 which protects the injured place against electromagnetic radiation and filter certain wavelengths from the radiation.

Topmost, on at least one surface of the tape 10, there is an adhesive 13 such as an adhesive layer which makes a detachable bond on the body surface. The adhesive 13 may also have been absorbed in the carrier layer 11. When placing kinesiology tape 10 on the back or around a limb, for example, it is stretched whereby it supports the attachment point when set in place. The tape 10 may be removed from its locality whereby, being elastic, it restores its original length,
In addition, a temperature rise takes place in the threads C1 and C2 and C1' and C2'. The threads C1, C2, C1', C2'... form a mesh or grid 20 which prevents waves higher than a particular wavelength of electromagnetic radiation from passing through the mesh 20.

The structure is well suited for sports injuries to speed up recovery but is also suitable for non-injured places to support muscles/tissues and to prevent injuries in advance.

Figure 2 shows electrically well conducting threads C1 and C2 knitted in the base material, such as cotton, of the kinesiology tape. In adjacent threads, they are wound in opposite directions S1 and S2, one clockwise and the other counterclockwise. Winding axes X1 and X2 are at a distance E from each other. E is advantageously in the range of 0.5 to 10 mm. The winding axes X1 and X2 are straight and parallel to each other. In adjacent windings the electromagnetic radiations cancel each other out. Plenty of heat is generated. The kinesiology tape 10 thus protects against electromagnetic radiation and produces heat to the place of the body being treated and supports the damaged place.

When the electrically conductive thread C1 is wound around its winding axis X1 in the winding direction S1, and the adjacent second thread C2 is wound around its winding axis X2 in the opposite winding direction S2, the electrically conductive threads C1, C2 wound in opposite directions are non-coaxial. When kinesiology tape has not got coaxial electrically conducting threads wound in opposite directions, the temperature rise in the electrically conductive threads can be kept low enough. The distance E between the winding axes X1 and X2 affects how much the electrically conductive threads warm up. When the distance E is small, the threads warm up more than when the distance E is larger. The electrically conductive thread C1 does not touch the adjacent second electrically conductive thread C2. There may be base material of the carrier layer between the electrically conductive thread C1 and the adjacent second electrically conductive thread C2.

So, between the electrically conducting threads C1 and C2, electrically non-conducting material may be woven. The electrically non-conducting material may be the material of the carrier layer 11. This electrically non-conducting material may also act as a structure cooler. On the other hand, the amount by which the structure warms up is affected by how far the electrically conductive threads C1 and C2 are from each other. The further away from each other the electrically conductive threads C1 and C2 are, the less warming takes place in the structure.

Often, a damaged place on the body swells up and gathers tissue fluid and waste materials. Electric voltage in near-by muscles increases to a high level.

The tape according to the invention supports the damaged place and removes excess electric voltage from the muscles. The waste materials can escape. The swelling of the area is reduced and the turning of the electromagnetic radiation caused by the muscle into managed heat in the mesh 20 treats the injured place, improves blood viscosity, and cancels out any excess electric charge formed in the muscle, and enhances the healing of the injury and removal of waste material from the injured place. Healing of the damaged body place speeds up.

The wound threads C1, C2, C1', C2' in Figures 2 and 3 form a grid, mesh or lattice 20 as in Figure 1, which prevents radiation higher than a particular wavelength from accessing the injured place.

Figure 3 shows an embodiment where the electrically conductive threads of kinesiology tape are wound, or coiled, and in which the winding or coiling directions of adjacent threads are different; one thread is wound clockwise, the adjacent thread counterclockwise. The threads may comprise in connection with them electrically non-conductive support threads that are wound in the winding direction of the electrically conductive threads along them. They provide the thread structure with rigidity as well as act as cooling threads and support threads.

The kinesiology tape 10 of Figure 3 is a rectangular structure as concerns its base material layer 11 which may be cotton, for example. There is an elastomer 12 joined in the base material layer to provide the tape with restoring elastic resilience.

It is also possible to set other electrically conductive additional materials in connection with the base material 11, such as an electrically conductive structure 14, such as a paint layer, a liquid containing electrically conductive particles, or electrically conductive gel.

Knitted on the surface of the base material layer in question there is an electrically well conducting first thread C1 wound on loops around its longitudinal axis C1, the winding direction S1 being clockwise.

Next to the electrically well conducting first thread C1 there is a second thread C2, which is also of an electrically well conducting material. It is wound or coiled counterclockwise S2. The winding axes X1 and X2 are at a distance E from each other, and the axes X1 and X2 are parallel to each other and therefore rectilinear. The distance E is advantageously in the range of 0.5 to 10 mm, and the thickness of the threads C1, C2; C1', C2' is advantageously in the range of 0.01 to 2 mm. To support the wound, electrically conductive threads, which may also be referred to as filaments or strings, there is a wound electrically non-conductive thread or a plurality of electrically non-conductive threads. Their task is to act as support threads and cooling threads, and furthermore one of their tasks is to maintain the distance E between the electrically conducting threads C1, C2; C1', C2'... Acting as support threads, they also act as cooling threads that prevent the temperature from rising too hot and keep the temperature under control. The temperature may be advantageously kept constant and slightly below the human body temperature. The material of the electrically conducting threads C1, C2; C1', C2' may be, for example, metal, carbon fibre, silver, or copper. When using metal, it repels bacteria and prevents their growth in the injured place.

In connection with the threads C1, C2; C1', C2', electrically non-conducting support threads may have been entwined to provide the total structure with rigidity. The support threads are electrically non-conducting.

This way, the winding directions alternate in adjacent threads. According to the invention the threads have been led as a continuous closed and grounded loop 200, 201 over the entire structure.

The threads form a mesh that prevents radiation of a particular wavelength from passing through the mesh 20. It therefore filters out certain wavelengths and this protects a person using the tape against electromagnetic radiation in two ways, so filtering out radiation and cancelling out electromagnetic fields in adjacent wound threads.

By introducing heat locally on the area of the injury, a treating effect is focused on the injury.

Electromagnetic fields in adjacent threads cancel each other out, and the electromagnetic radiation transforms into heat.

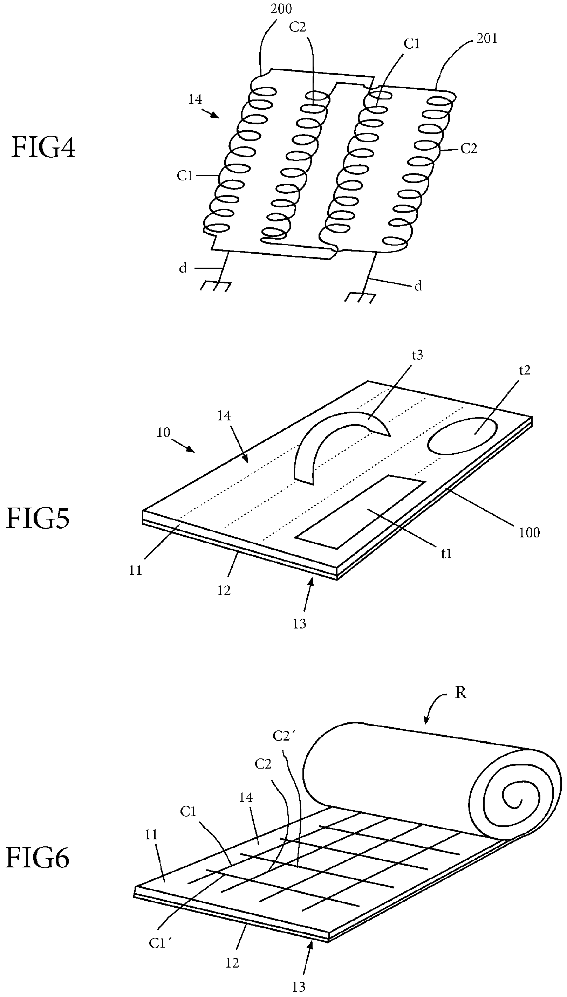

Figure 4 shows how the threads C1 and C2 are introduced as closed loops 200, 201. The loops 200, 201 and therefore wires C1 and C2 are grounded by ground wires d.

This way, the tape allows the soothing kinesiology tape 10 to be brought in contact with the injury, whereby the treatment has a heat effect as well as an effect treating and supporting the tissue. By stretching the kinesiology tape 10, an injured ankle, for example, may be well supported and compressed. The introduction of heat promotes the shifting away of tissue fluid that has gathered in the injured area and helps the tissue recover.

The mesh formed with the threads filters out damaging wavelengths from room radiation, and consequently the ill effects of the radiation in question to the body are eliminated.

The winding directions of the threads C1, C2 are illustrated by arrows S1 and S2.

The thickness of the threads C1 and C2, that is, the cross-section O, is in the range of 0.01 to 2 mm, and the material used is, for example, carbon fibre, silver thread, or copper thread.

Figure 5 shows the preferred sheet embodiment of the tape 10. There are rectangular sheets one upon the other, and each has cutting lines t1, t2, ... for injuries of different kinds. The structure is well suited for the post office to transport.

Figure 5 shows kinesiology tape material as sheets 100.

In Figure 6, a roll embodiment R of the kinesiology tape 10 is shown. The kinesiology tape 10 is wound as a roll R and may be unwound from the roll R.

Those skilled in the art will find it obvious that, as technology advances, the basic idea of the invention may be implemented in many different ways. The invention and its embodiments are thus not restricted to the above-described examples but may vary within the scope of the claims.

## Claims

1. A kinesiology tape (10) which comprises a carrier layer (11), an elastomer (12) connected to the carrier layer (11) and providing the tape with elasticity which after stretching is restored, the kinesiology tape (10) comprising an adhesive (13) on the surface of the carrier layer (11), by means of which the tape is removably fastened to the skin, and the kinesiology tape (10) comprising an electrically conductive structure (14), **characterized in that** the electrically conductive structure (14) is formed of electrically conductive threads (C1, C2) placed side-by-side, that a first electrically conductive thread (C1) is wound or coiled around its winding axis (X1) in a winding direction (S1) and an adjacent second thread (C2) is wound or coiled around its winding axis (X2) in the opposite winding direction (S2), that is, one set of threads (C1) is wound clockwise (S1) and the other set of threads is wound counterclockwise (S2), and that the first threads (C1) wound clockwise are introduced as a first closed loop (200), and the second threads (C2) wound counterclockwise are introduced as a second closed loop (201), whereby one set of threads is wound clockwise and the adjacent threads in relation thereto counterclockwise, whereby the electromagnetic radiations in adjacent wound threads cancel each other out and heat is generated.

2. A kinesiology tape as claimed in claim 1, **characterized in that** one set of threads (C1, C2) is placed rectilinearly vertically and, in relation to them, a second set of electrically conductive threads (C1', C2') is placed horizontally so that they form a mesh or grid (20) which prevents electromagnetic radiation exceeding a particular wavelength from passing through the lattice (20).

3. A kinesiology tape (10) as claimed in claim 1 or 2, **characterized in that** the winding axes (X1, X2) of the threads (C1, C2; C1', C2') are parallel to each other, and the distance (E) between them is in the range of 0.5 to 10 mm, and the thickness of the threads (C1, C2; C1', C2') in their cross section is in the range of 0.01 to 2 mm, and that the material of the threads (C1, C2; C1', C2') is an electrically conductive material such as metal, silver, carbon fibre, or copper.

4. A kinesiology tape (10) as claimed in any one of the preceding claims, **characterized in that** between the first electrically conductive threads (C1) and second electrically conductive threads (C2) there is electrically non-conductive material.

5. A kinesiology tape as claimed in claim 4, **characterized in that** the electrically non-conductive material is the carrier layer material.

6. A kinesiology tape (10) as claimed in any one of the preceding claims, **characterized in that** the electrically conductive threads (C1, C2) are grounded, and **in that** the closed loops formed of the electrically conductive threads (C1, C2) comprises a grounding wire (d).

7. A kinesiology tape (10) as claimed in any one of the preceding claims, **characterized in that** the wound electrically conductive threads (C1, C2; C1', C2') each comprise in connection thereof an electrically non-conductive support thread whose winding direction counterclockwise or clockwise is the same as that of the electrically conductive thread (C1, C2; C1', C2') supported, whereby the support threads act not only as support threads but also as cooling threads.

8. A kinesiology tape (10) as claimed in any one of the preceding claims, **characterized in that** the kinesiology tape (10) is formed of sheets (100) that are one upon the other, making them easy to mail.

9. A kinesiology tape (10) as claimed in claim 8, **characterized in that** the sheet comprises, on the surface of the tape, cutting lines (t1, t2, t3...) to cut off tape appropriate for different uses from the kinesiology tape sheet (100).

10. A kinesiology tape (10) as claimed in any one of claims 1 to 7, **characterized in that** the kinesiology tape (10) is on a roll (R).

## Patentansprüche

1. Kinesiologieband (10), das eine Trägerschicht (11), ein Elastomer (12), das mit der Trägerschicht (11) verbunden ist und dem Band Elastizität verleiht, die nach dem Dehnen wiederhergestellt wird, umfasst, wobei das Kinesiologieband (10) einen Klebstoff (13) auf der Oberfläche der Trägerschicht (11) umfasst , mit dem das Band abnehmbar auf der Haut befestigt wird, und wobei das Kinesiologieband (10) eine elektrisch leitfähige Struktur (14) umfasst, **dadurch gekennzeichnet, dass** die elektrisch leitfähige Struktur (14) aus elektrisch leitfähigen Fäden (C1, C2) gebildet ist, die nebeneinander angeordnet sind, dass ein erster elektrisch leitfähiger Faden (C1) in einer Wickelrichtung (S1) um seine Wickelachse (X1) gewickelt oder gerollt ist und ein benachbarter zweiter Faden (C2) in der entgegengesetzten Wickelrichtung (S2) um seine Wickelachse (X2) gewickelt oder gerollt ist, d.h. dass ein Satz von Fäden (C1) im Uhrzeigersinn (S1) und der andere Satz von Fäden gegen den Uhrzeigersinn (S2) gewickelt ist, und dass die ersten Fäden (C1), die im Uhrzeigersinn gewickelt sind, als eine erste geschlossene Schleife (200) eingeführt werden und die zweiten Fäden (C2), die gegen den Uhrzeigersinn gewickelt sind, als eine zweite geschlossene Schleife (201) eingeführt werden, wodurch ein Satz von Fäden im Uhrzeigersinn und die benachbarten Fäden in Bezug dazu gegen den Uhrzeigersinn gewickelt werden, wodurch die elektromagnetischen Strahlungen in benachbarten gewickelten Fäden sich gegenseitig aufheben und Wärme erzeugt wird.

2. Kinesiologieband nach Anspruch 1, **dadurch gekennzeichnet, dass** ein Satz von Fäden (C1, C2) geradlinig vertikal angeordnet ist und in Bezug auf diese ein zweiter Satz von elektrisch leitfähigen Fäden (C1', C2') horizontal angeordnet ist, so dass sie eine Masche oder ein Netz (20) bilden, das verhindert, dass elektromagnetische Strahlung, die eine bestimmte Wellenlänge überschreitet, das Gitter (20) durchdringt.

3. Kinesiologieband (10) nach Anspruch 1 oder 2, **dadurch gekennzeichnet, dass** die Wickelachsen (X1, X2) der Fäden (C1, C2; C1', C2') parallel zueinander verlaufen und der Abstand (E) zwischen ihnen im Bereich von 0,5 bis 10 mm liegt, und die Dicke der Fäden (C1, C2; C1', C2') in ihrem Querschnitt im Bereich von 0,01 bis 2 mm liegt, und dass das Material der Fäden (C1, C2; C1', C2') ein elektrisch leitfähiges Material wie Metall, Silber, Kohlefaser oder Kupfer ist.

4. Kinesiologieband (10) nach einem der vorstehenden Ansprüche, **dadurch gekennzeichnet, dass** sich zwischen den ersten elektrisch leitfähigen Fäden (C1) und den zweiten elektrisch leitfähigen Fäden (C2) elektrisch nicht leitfähiges Material befindet.

5. Kinesiologieband nach Anspruch 4, **dadurch gekennzeichnet, dass** das elektrisch nicht leitfähige Material das Trägerschichtmaterial ist.

6. Kinesiologieband (10) nach einem der vorstehenden Ansprüche, **dadurch gekennzeichnet, dass** die elektrisch leitfähigen Fäden (C1, C2) geerdet sind und dass die aus den elektrisch leitfähigen Fäden (C1, C2) gebildeten geschlossenen Schleifen einen Erdungsdraht (d) umfassen.

7. Kinesiologieband (10) nach einem der vorstehenden Ansprüche, **dadurch gekennzeichnet, dass** die gewickelten elektrisch leitfähigen Fäden (C1, C2; C1', C2') jeweils in Verbindung damit einen elektrisch nicht leitfähigen Stützfaden umfassen, dessen Wickelrichtung entgegen dem Uhrzeigersinn oder im Uhrzeigersinn die gleiche ist wie die des gestützten elektrisch leitfähigen Fadens (C1, C2; C1', C2'), wodurch die Stützfäden nicht nur als Stützfäden, sondern auch als Kühlfäden wirken.

8. Kinesiologieband (10) nach einem der vorstehenden Ansprüche, **dadurch gekennzeichnet, dass** das Kinesiologieband (10) aus übereinander liegenden Blättern (100) gebildet ist, die leicht zu versenden sind.

9. Kinesiologieband (10) nach Anspruch 8, **dadurch gekennzeichnet, dass** das Blatt auf der Oberfläche des Bandes Schnittlinien (t1, t2, t3...) aufweist, um von dem Kinesiologieband-Blatt (100) ein für verschiedene Zwecke geeignetes Band abzuschneiden.

10. Kinesiologieband (10) nach einem der Ansprüche 1 bis 7, **dadurch gekennzeichnet, dass** das Kinesiologieband (10) auf einer Rolle (R) ist.

## Revendications

1. Bande de kinésiologie (10) qui comprend une couche de support (11), un élastomère (12) connecté à la couche de support (11) et dotant la bande d'une élasticité qui est rétablie après étirement, la bande de kinésiologie (10) comprenant un adhésif (13) sur la surface de la couche de support (11) au moyen duquel la bande est fixée à la peau de façon amovible, et la bande de kinésiologie (10) comprenant une structure électriquement conductrice (14), **caractérisée en ce que** la structure électriquement conductrice (14) est formée de fils électriquement conducteurs (C1, C2) placés côte à côte, **en ce qu'**un premier fil électriquement conducteur (C1) est enroulé ou bobiné autour de son axe d'enroulement (X1) dans une direction d'enroulement (S1) et un deuxième fil adjacent (C2) est enroulé ou bobiné autour de son axe d'enroulement (X2) dans la direction d'enroulement opposée (S2), en d'autres termes un jeu de fils (C1) est enroulé dans le sens horaire (S1) et l'autre jeu de fils est enroulé dans le sens contra-horaire (S2), et **en ce que** les premiers fils (C1) enroulés dans le sens horaire sont introduits sous la forme d'une première boucle fermée (200), et les deuxièmes fils (C2) enroulés dans le sens contra-horaire sont introduits sous la forme d'une deuxième boucle fermée (201), moyennant quoi un jeu de fils est enroulé dans le sens horaire et les fils adjacents en relation avec celui-ci dans le sens contra-horaire, moyennant quoi les rayonnements électromagnétiques dans des fils enroulés adjacents s'annulent mutuellement et de la chaleur est générée.

2. Bande de kinésiologie selon la revendication 1, **caractérisée en ce qu'**un jeu de fils (C1, C2) est placé de façon rectiligne verticalement et, en relation avec celui-ci, un deuxième jeu de fils électriquement conducteurs (C1', C2') est placé horizontalement de façon à former un treillis ou une grille (20) qui empêche le rayonnement électromagnétique dépassant une longueur d'onde particulière de passer à travers le réseau (20).

3. Bande de kinésiologie (10) selon la revendication 1 ou 2, **caractérisée en ce que** les axes d'enroulement (X1, X2) des fils (C1, C2 ; C1', C2') sont mutuellement parallèles, et la distance (E) entre eux est située dans la plage allant de 0,50 à 10 mm, et l'épaisseur des fils (C1, C2 ; C1', C2') dans leur section transversale est située dans la plage allant de 0,01 à 2 mm, et **en ce que** le matériau des fils (C1, C2 ; C1', C2') est un matériau électriquement conducteur tel qu'un métal, l'argent, les fibres de carbone, ou le cuivre.

4. Bande de kinésiologie (10) selon l'une quelconque des revendications précédentes, **caractérisée en ce qu'**il y a un matériau électriquement non conducteur entre les premiers fils électriquement conducteurs (C1) et les deuxièmes fils électriquement conducteurs (C2).

5. Bande de kinésiologie selon la revendication 4, **caractérisée en ce que** le matériau électriquement non conducteur est le matériau de couche de support.

6. Bande de kinésiologie (10) selon l'une quelconque des revendications précédentes, **caractérisée en ce que** les fils électriquement conducteurs (C1, C2) sont mis à la masse, et **en ce que** les boucles fermées formées des fils électriquement conducteurs (C1, C2) comprennent un fil de mise à la masse (d).

7. Bande de kinésiologie (10) selon l'une quelconque des revendications précédentes, **caractérisée en ce que** chacun des fils électriquement conducteurs enroulés (C1, C2 ; C1', C2') comprend, en connexion avec celui-ci, un fil de support électriquement non conducteur dont la direction d'enroulement contra-horaire ou horaire est la même que celle du fil électriquement conducteur (C1, C2 ; C1', C2') supporté, moyennant quoi les fils de support agissent non seulement comme des fils de support mais aussi comme des fils de refroidissement.

8. Bande de kinésiologie (10) selon l'une quelconque des revendications précédentes, **caractérisée en ce que** la bande de kinésiologie (10) est formée de feuilles (100) qui sont les unes sur les autres, ce qui les rend faciles à poster.

9. Bande de kinésiologie (10) selon la revendication 8, **caractérisée en ce que** la feuille comprend, sur la surface de la bande, des lignes de découpe (t1, t2, t3 ...) pour découper une bande à partir de la feuille de bande de kinésiologie (100) de façon appropriée pour différentes utilisations.

10. Bande de kinésiologie (10) selon l'une quelconque des revendications 1 à 7, **caractérisée en ce que** la bande de kinésiologie (10) est sur un rouleau (R).
